# EUROPEAN PATENT APPLICATION

(11) **EP 4 109 491 A2**
(43) Date of publication of application: **28.12.2022**
(21) Application number: 22175633.1
(22) Date of filing: 23.06.2022
(51) Int. Cl.: H01J 61/073, A61L 2/10, H01J 61/35, H01J 61/40, H01J 65/04

(54) **A NOVEL ULTRAVIOLET LAMP TUBE**

(30) Priority: 22.06.2021 CN 202110689195; 03.09.2021 CN 202112123367
(71) Applicant: Langsim Optoelectronic Technologies (Guangdong) Limited, Nanhai, Foshan Guangdong (CN)
(72) Inventor: Li, Jiawei, FOSHAN (CN)
(74) Representative: Díaz de Bustamante y Terminel, Isidro

(57) **Abstract**

The present application discloses a novel ultraviolet lamp tube, comprising an outer tube 1; an inner tube 2 coaxially installed inside the outer tube; an outer electrode 4 embedded on the outer tube; and an inner electrode 3 installed inside the inner tube; wherein the outer surface of the inner electrode or inner tube is plated with a clutter waves coating 31 or an inner tube coating 21 that can reflect a desired wavelength band; and the outer sidewall of the outer tube is plated with a single-wavelength band coating 11 that can transmit a single-wavelength band. The coating method is used for wave filtering to ensure monochromaticity of the light emitted by the light source and greatly reduce the volume of the ultraviolet lamp tube. At the same time, the ultraviolet lamp can be in a cylindrical shape to effectively increase the beam angle of the ultraviolet lamp and effectively improve the luminous efficiency of the ultraviolet lamp.

## Description

### CROSS-REFERENCE TO RELATED APPLICATION

This application claims priority to Chinese Patent Application o. 202110689195.5, filed on June 22, 2021, and Chinese Patent Application No. 202122123367.0, filed on September 3, 2021, with the China National Intellectual Property Administration, the disclosures of both of which are incorporated herein by reference in their entirety.

### FIELD OF THE DISCLOSURE

The present invention relates to the technical field of ultraviolet lamps, in particular, to a novel ultraviolet lamp tube with a high luminous efficiency.

### BACKGROUND OF THE DISCLOSURE

The existing ultraviolet lamp tube uses a light-filtering plate to filter the wavelength band that is harmful to the human body, but the structure still has drawbacks such as a small beam angle, non-uniform light source, and low luminous efficiency, thereby reducing the luminous efficiency of the entire ultraviolet lamp; in addition, due to the arrangement of the light-filtering plate, the volume of the existing UV lamp tube is relatively large. To this end, we provide a novel UV lamp tube with a high luminous efficiency to solve the above-mentioned problems.

### SUMMARY OF THE DISCLOSURE

In view of the defects of the existing ultraviolet lamps in the above-mentioned background technique, such as small beam angle, non-singular light source, and poor luminous efficiency, the present disclosure provides an ultraviolet lamp tube with a high luminous efficiency to overcome these drawbacks.

In one aspect, the present disclosure provides a novel ultraviolet lamp tube with a high luminous efficiency, comprising: an outer tube; an inner tube coaxially installed inside the outer tube; an outer electrode embedded on the outer tube; an inner electrode installed inside the inner tube; the outer surface of the inner electrode is plated with a clutter waves coating; the outer sidewall of the outer tube is plated with a single-wavelength band coating.

In one embodiment, sometimes preferred, the single-wavelength band coating can transmit only a single-wavelength band out of the outer tube.

In one embodiment, sometimes preferred, the clutter waves coating transmits the clutter waves that cannot be transmitted outside the outer tube to the inner electrode and is absorbed by the inner electrode, and at the same time the clutter waves coating reflects the usable single wavelength band to the single wavelength band coating for transmitting outside of the outer tube.

In one aspect, the present disclosure provides a novel ultraviolet lamp tube with a high luminous efficiency, comprising: an outer tube; an inner tube coaxially installed inside the outer tube; an outer electrode embedded on the outer tube; an inner electrode installed inside the inner tube; the outer surface of the inner tube is plated with a coating (the "inner tube coating") that can reflect a desired wavelength band; and the outer sidewall of the outer tube is plated with a single-wavelength band coating capable of transmitting a single-wavelength band.

In one embodiment, sometimes preferred, the single-wavelength band coating can transmit only a single-wavelength band out of the outer tube.

In one embodiment, sometimes preferred, the inner tube coating transmits the clutterwaves that cannot be transmitted out of the outer tube through the coating layer to the inner tube, which are transmitted through the inner tube to the inner electrode and will be absorbed by the inner electrode; and at the same time the inner tube coating reflects the useful single wavelength band to the outer tube and transmits it out.

In one embodiment, sometimes preferred, the single-wavelength band coating and the inner tube coating are both light-filter coatings.

In another aspect, the present disclosure provides a device comprising a novel ultraviolet lamp tube according to any embodiment disclosed herein, for example, while not intended to be limiting, the novel ultraviolet lamp tube can be used in a disinfection device for antibacterial and/or antiviral applications, preferably in a space occupied by humans and/or animals, including but not limited to domestic pets or farm-raised animals, such as cats, dogs, monkeys, horses, pigs, cattles, poultry, or the like.

Among other advantages, through plating the outer surface of the electrode or the inner tube with a clutter waves coating or the inner tube coating capable of transmitting the clutter waves, and plating the outside wall of the outer tube with a single-wavelength band coating capable of transmitting a desired wavelength band, the present invention filters waves via the plating methods, which can ensure monochromaticity of the light from the light source. Specifically, the single-wavelength band coating can only transmit the single wavelength band out of the outer tube to effectively ensure monochromaticity of the light from the light source, such as a single wavelength of 207 nm or 222 nm or 250 nm or 308 nm, and so on, and the clutter waves coating or the inner tube coating will transmit the clutter waves that cannot be transmitted outside the outer tube through the coating to the inner electrode, thus absorbed by the inner electrode; and meanwhile the inner tube coating reflects the useful single-wavelength band to the outer tube and transmits it out. In addition, by means of coating, light-filtering plates are not needed; thus, the volume of the UV lamp tube can be greatly reduced, and the UV lamp tube can be designed in a cylindrical shape to effectively increase the beam angle of the UV lamp tube. At the same time, the clutter waves coating reflects the usable single wavelength band to the single wavelength band coating and transmits it to the outside of the outer tube, thereby effectively improving the luminous efficiency of the ultraviolet lamp tube.

Other aspects or advantages of the disclosure will be better appreciated in view of the following drawings, detailed description, examples, and claims.

### BRIEF DESCRIPTION OF THE DRAWINGS

FIG. 1 is the structural schematic diagram of the present invention.
FIGs. 2A and 2B (collectively "FIG. 2") are cross-sectional views of the exemplified ultraviolet lamp tubes.
FIGs. 3A and 3B (collectively "FIG. 3") are illustrative diagrams of the paths of different wavelength bands in the exemplified ultraviolet lamp tubes.

In the figures: 1-outer tube; 2-inner tube; 3-inner electrode; 4-outer electrode; 11-single wavelength coating; 31- clutter waves coating; and 21-inner tube coating.

### DETAILED DESCRIPTION OF CERTAIN EMBODIMENTS

The present invention will now be described in further detail with reference to the accompanying drawings. These drawings are all simplified schematic diagrams, and only illustrate the basic structure of the present invention in a schematic manner, so they only show the structures related to the present invention.

In the present disclosure, it should be noted that the orientation or positional relationships indicated by the terms "inside", "outside", "between", or the like, are based on the orientation or positional relationships shown in the accompanying drawings. They only serve for the convenience of describing the present invention and simplifying the description, rather than indicate or imply that the referred device or element must have a specific orientation, or be constructed and operated in a specific orientation, so they should not be construed to be limiting on the present invention.

In addition, unless otherwise expressly specified and defined, the terms "install", "wind", and "connect" should be understood in a broad sense, for example, it can be a fixed connection, a detachable connection, or an integral connection; it can be a mechanical connection or an electrical connection; it can be a direct connection or an indirect connection through an intermediate medium; and it can be internal communication between two components of a unit. For those of ordinary skill in the art, the specific meanings of the above terms in the present disclosure can be understood under the specific circumstances.

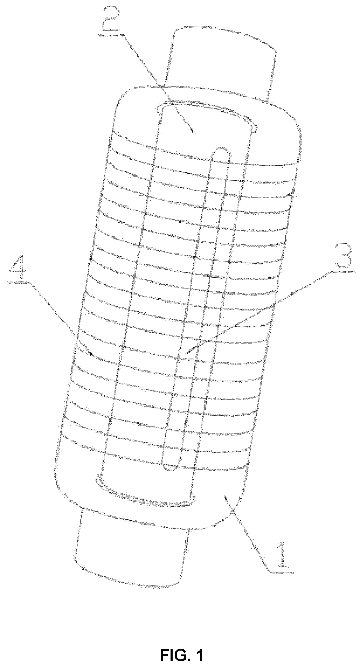

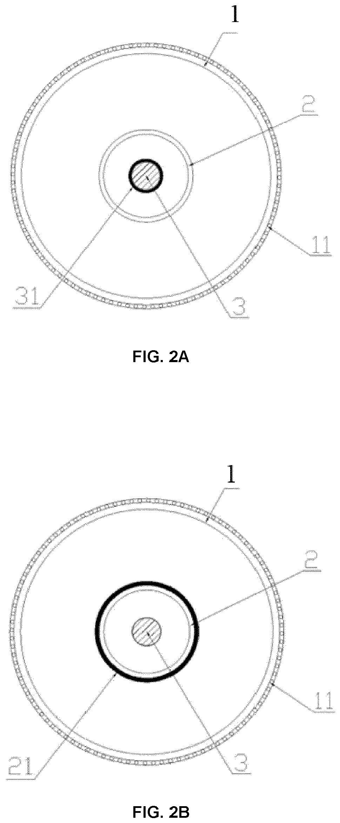

As shown in FIGs. 1-2, a novel ultraviolet lamp tube with a high luminous efficiency, comprising an outer tube 1; an inner tube 2 is coaxially installed inside the outer tube 1; the outer tube 1 is embedded with an outer electrode 4; the inner tube 2 is provided with an inner electrode 3; the outer surface of the inner electrode 3 is plated with a clutter waves coating 31 (FIG. 2A), or the inner tube 2 is plated with a coating (the "inner tube coating") 21 (FIG. 2B) capable of reflecting a desired wavelength band; the outside wall of the outer tube 1 is coated with a single-wavelength band coating 11, which is capable of transmitting a single-wavelength band.

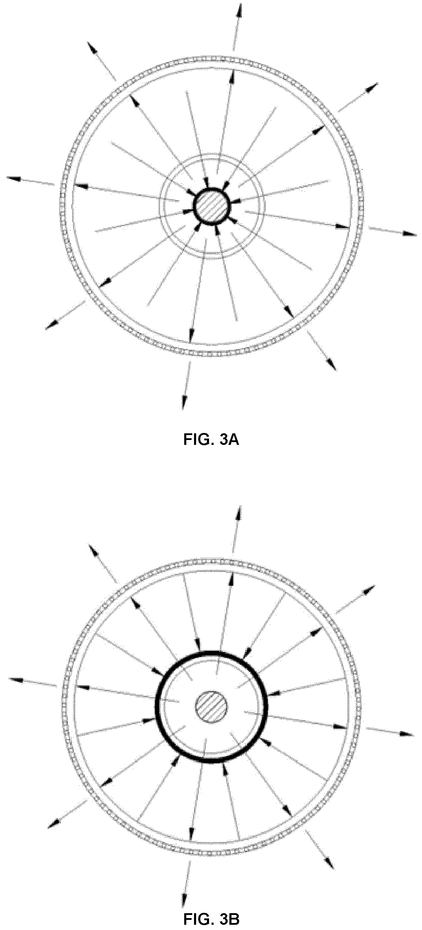

Referring to FIG. 3, specifically, the single-wavelength band coating 11 can transmit only a wavelength band of a single wavelength out of the outer tube 1; the clutter waves coating 31 transmits the clutter waves that cannot be transmitted through the coating out of the outer tube 1 to the inner electrode 3, or the inner tube coating 21 transmits the clutter waves to the inner tube 2, through which the clutter wavers are transmitted to the inner electrode 3 and absorbed by the inner electrode 3 , and at the same time, the clutter waves coating 31 reflects the useful single wavelength band to the single wavelength coating 11 for transmitting to the outside of the outer tube 1 (FIG. 3A); or the inner tube coating 21 reflects the useful single wavelength band to the outer tube 1 and transmits it out (FIG. 3B).

In the present disclosure, the single-wavelength band coating and the inner tube coating are both light-filter coatings.

The present invention, through plating the outer surface of the electrode 3 or inner tube 2 with a clutter waves coating 31 or inner tube coating 21 capable of transmitting clutter waves, and plating the outside wall of the outer tube 1 with a single-wavelength band coating 11 capable of transmitting a desired wavelength band, filters light waves through the method of plating, which can ensure monochromaticity of the light from the light source, that is, the single-wavelength band coating 11 can transmit only the single wavelength band out of the outer tube 1 to effectively ensure the single wavelength of the light from the light source, such as 207 nm, 222 nm, 250 nm, or 308 nm, or the like, and the clutter waves coating 31 or the inner tube coating 21 will transmit the clutter waves that cannot be transmitted outside the outer tube 1 to the inner electrode 3, and is absorbed by the inner electrode 3. In addition, the volume of the UV lamp tube can be greatly reduced by means of coating, and the UV lamp tube can be designed in a cylindrical shape to effectively increase the beam angle of the UV lamp tube. At the same time, the clutter waves coating reflects the usable single wavelength band to the single wavelength band coating and transmits it to the outside of the outer tube, or the inner tube coating reflects the useful single wavelength band to the outer tube and transmits it out, thereby effectively improving the luminous efficiency of the ultraviolet lamp tube.

In the description of this specification, the reference terms such as "one embodiment", "some embodiments", "exemplary embodiment", "example", "specific example" or "some examples", or the like, mean that a particular feature, structure, material, or characteristic described in such embodiment or an example is included in at least one embodiment or illustrative example of the present invention. In this application, schematic representations of the above terms do not necessarily refer to the same embodiment or example. Furthermore, the particular features, structures, materials or characteristics described may be combined in a suitable manner in any one or more embodiments or examples.

The above has shown and described the basic principles and major features and advantages of the present invention, and it will be apparent to those skilled in the art that the present invention is not limited to the details of the above-described exemplary embodiments, and that without departing from the spirit or essential characteristics of the present invention, the present invention can be implemented in other specific forms. Therefore, the embodiments are to be regarded in all respects as illustrative and non-limiting, and the scope of the invention is defined by the appended claims rather than the foregoing description, and it is therefore intended to embrace within the present invention all changes or modifications that come within the meaning and range or equivalents of the claims. Any reference symbols or numbers to the drawings in the claims shall not be construed as limiting the involved claims.

In addition, it should be understood that although this invention is described in terms of embodiments, it is not the case that each embodiment only includes an independent technical solution, and this description in the specification is only for the purpose of clarity, and those skilled in the art should take the specification as a whole, since the technical solutions in each embodiment can also be appropriately combined to form other embodiments that can be understood by those skilled in the art.

## Claims

1. An ultraviolet lamp tub, comprising an outer tube; an inner tube coaxially installed inside the outer tube; an outer electrode embedded on the outer tube; an inner electrode installed inside the inner tube; wherein outer surface of the inner electrode is plated with a clutter waves coating; and outer sidewall of the outer tube is plated with a single-wavelength band coating.

2. The ultraviolet lamp tube of claim 1, wherein the single-wavelength coating can only transmit a single-wavelength band out of the outer tube.

3. The ultraviolet lamp tube of claim 2, wherein the clutter waves coating transmits all the clutter waves that cannot be transmitted out of the outer tube to the inner electrode and is absorbed by the inner electrode; and wherein, at the same time, the clutter waves coating reflects the useful single-wavelength band to the single-wavelength band coating for transmitting it outside of the outer tube.

4. An ultraviolet lamp tub, comprising an outertube; an inner tube coaxially installed inside the outer tube; an outer electrode embedded on the outer tube; an inner electrode installed inside the inner tube; wherein outer surface of the inner tube is plated with an inner tube coating capable of reflecting a desired wavelength band; and outer sidewall of the outer tube is plated with a single-wavelength band coating capable of transmitting a single-wavelenth band.

5. The ultraviolet lamp tube of claim 4, wherein the single-wavelength coating can only transmit a single-wavelength band out of the outer tube.

6. The ultraviolet lamp tube of claim 5, wherein the inner tube coating transmits clutter waves that cannot be transmitted out of the outer tube through the coating layer to the inner tube, wherein the reflected clutter waves are transmitted through the inner tube to the inner electrode and absorbed by the inner electrode; and wherein, at the same time, the inner tube coating reflects the useful single wavelength band to the outer tube and transmits it out.

7. The ultraviolet lamp tube of claim 6, wherein the single-wavelength band coating and the inner tube coating are both light-filter coatings.

8. A device comprising an ultraviolet lamp tube of claim 1.

9. The device of claim 8, which is a disinfection device.

10. A device comprising an ultraviolet lamp tube of claim 4.

11. The device of claim 10, which is a disinfection device.
